Europäisches Patentamt

European Patent Office

Office européen des brevets

(19)

(11) Veröffentlichungsnummer: **0 078 820**
**B1**

(12) **EUROPÄISCHE PATENTSCHRIFT**

(45) Veröffentlichungstag der Patentschrift:
**09.12.87**

(51) Int. Cl.⁴: **C 08 B 37/16**

(21) Anmeldenummer: **82901423.2**

(22) Anmeldetag: **12.05.82**

(86) Internationale Anmeldenummer:
**PCT/HU 82/00024**

(87) Internationale Veröffentlichungsnummer:
**WO 82/04052 (25.11.82 Gazette 82/28)**

(54) INKLUSIONSCOMPLEXE VON N-(1-PHENYLÄTHYL)-3,3-DIPHENYLPROPYLAMIN BZW. DESSEN HYDROCHLORID MIT CYCLODEXTRIN, VERFAHREN ZUR HERSTELLUNG DIESER INKLUSIONSKOMPLEXE SOWIE DIESE INKLUSIONSKOMPLEXE ENTHALTENDE PHARMAZEUTISCHE PRÄPARATE.

(30) Priorität: **12.05.81 HU 128681**

(43) Veröffentlichungstag der Anmeldung:
**18.05.83 Patentblatt 83/20**

(45) Bekanntmachung des Hinweises auf die Patenterteilung:
**09.12.87 Patentblatt 87/50**

(84) Benannte Vertragsstaaten:
**AT CH DE FR GB LI**

(73) Patentinhaber: **CHINOIN Gyogyszer és Vegyészeti Termékek Gyára RT., To utca 1-5, H-1045 Budapest IV (HU)**

(72) Erfinder: **SZEJTLI, József, Dr., Endrödi S. u. 38-40, H-1026 Budapest (HU)**
Erfinder: **STADLER, Agnes, Dr., Nepstadion ut 18, H-1143 Budapest (HU)**
Erfinder: **VIKMON, Mária, Dr., Endrödi S. u. 24, H-1026 Budapest (HU)**
Erfinder: **KORBONITS, Dezsö, Dr., Vérhalom ut 27/d, H-1025 Budapest (HU)**
Erfinder: **VIRAG, Sándor, Dr., Sallai I. u. 29/a, H-1136 Budapest (HU)**
Erfinder: **TURCSAN, István, Örs vezer tér 11, H-1148 Budapest (HU)**
Erfinder: **KISS, Pál, Dr., Vetés ut 82, H-1046 Budapest (HU)**

(74) Vertreter: **Lotterhos, Hans Walter, Dr.-Ing., Lichtensteinstrasse 3, D-6000 Frankfurt am Main 1 (DE)**

(56) Entgegenhaltungen:
EP - A - 0 018 773
SU - A - 915 807
CHEMICAL ABSTRACTS, Band 88, Nr. 14, 3. April 1978, Seite 93, Zusammenfassung 91364c, COLUMBUS, OHIO (US),
"Chemical and Pharmaceutical Bulletin" volume 23, no. 7, published on July 1975, (Tokyo, Japan), Kaneto Uekama et al. "Inclusion Complexes of Cinnamic Acids with Cyclodextrins. Mode of Inclusion in Aqueous Solution" 1421-1430

(56) Entgegenhaltungen: (Fortsetzung)
"Chemical and Pharmaceutical Bulletin" volume 23, no. 12, published on December 1975 (Tokyo, Japan), Masashi Kurozumi et al. "Inclusion Compounds of Non-Steroidal Antiinflammatory and other Slightly Water Soluble Drugs with - and - Cyclodextrins in Powdered Form", 3062-3068

EP 0 078 820 B1

# Beschreibung

Technisches Gebiet

Die Erfindung betrifft Inklusionskomplexe von N-(1-Phenyläthyl)-3,3-diphenylpropylamin bzw. dessen Hydrochlorid mit Cyclodextrin sowie Verfahren zur Herstellung derartiger Inklusionskomplexe. Das Verfahren besteht darin, dass man die N-(1-Phenyläthyl)-3,3-diphenylproylamin-Base bzw. deren Hydrochlorid in wässrigem und/oder äthanolischem Medium unter Rühren bei 4–60 °C mit Cyclodextrin umsetzt und gewünschtenfalls den mit der Base erhaltenen Cyclodextrin-Komplex mit Salzsäure behandelt.

Stand der Technik

N-(Phenyläthyl)-3,3-diphenylpropylamin (nachfolgend Phendilin genannt) ist ein koronardilatierender, Calcium-antagonistischer Wirkstoff, dessen Herstellung in der HU-PS 150 534 beschrieben ist. Die Verbindung stellt eine ölige Flüssigkeit dar, deren Hydrochloridsalz unter der Handelsbezeichnung Sensit als Medikament bekannt ist. Eine feste Medikamentenform kann nur aus dem Hydrochlorid hergestellt werden. Phendilin-Hydrochlorid ist mit Wasser nur schlecht benetzbar, da der Stoff ausgesprochen hydrophob ist. Die Resorptonsgeschwindigkeit des Phendilins sowie das Mass der Resorption sind ungenügend.

Darstellung der Erfindung

Ziel der Erfindung war, die Entwicklung eines Produktes und eines Verfahrens zur Herstellung eines Produktes, wodurch Phendilin und dessen Hydrochlorid in eine schneller und besser lösliche Form gebracht und dadurch die Geschwindigkeit und das Mass der Resorption wesentlich verbessert werden kann.

Es wurde gefunden, dass Phendilin oder dessen Hydrochlorid nach Überführung in einen Cyclodextrin-Inklusionskomplex bei einem der Magensäure entsprechenden pH-Wert und Temperatur viel schneller und in grösserer Konzentration in Lösung geht, als das ursprüngliche Molekül.

Bekanntlich sind die Cyclodextrinmoleküle zylinderförmig und weisen eine apolare innere Oberfläche auf, was sie dazu befähigt, hydrophobe Moleküle in Form von Inklusionskomplexen zu binden. Ein solches hydrophobes Molekül kann Phendilin oder dessen Hydrochlorid sein, beide sind in Wasser sehr schwer löslich.

Es wurde gefunden, dass bei der Umsetzung von Phendilin mit Cyclodextrinen zwei Cyclodextrinmoleküle mit einem Phendilinmolekül einen Inklusionskomplex bilden, der überraschenderweise in einer dem pH-Wert der Magensäure entsprechenden wässrigen Salzsäurelösung eines der Cyclodextrinmoleküle verliert und mit grosser Geschwindigkeit in einen molekular-dispersen Zustand übergeht. Auf diese Weise kann eine wesentlich bessere Löslichkeit – und als Folge davon – auch eine bessere biologische Anwendbarkeit erzielt werden.

Zur Durchführung des Verfahrens der Erfindung werden N-(1-Phenyläthyl)-3,3-diphenylpropyl-amin-Base oder deren Hydrochlorid im wässrigen und/oder äthanolischen Medium bei 4–60 °C unter intensivem Rühren mit Cyclodextrin im Verhältnis von 1–3 mMol Cyclodextrin pro 1 mMol freie Base oder Hydrochlorid umgesetzt.

Zur Herstellung des Hydrochlorid-Cyclodextrin-Komplexes kann entweder die Umsetzung der freien Base mit Cyclodextrin in Gegenwart von Salzsäure durchgeführt werden, wobei die Komplexbildung mit dem in situ gebildeten Hydrochlorid des N-(1-Phenyläthyl)-3,3-diphenylpropylamins stattfindet, oder der bei der Umsetzung mit der freien Base erhaltene Cyclodextrininklusionskomplex nachträglich durch Behandeln mit Salzsäure in den Hydrochlorid-Cyclodextrin-Komplex übergeführt werden.

Als Cyclodextrin wird vorzugsweise α-, β- oder γ-Cyclodextrin verwendet.

Bei Reaktionsdurchführung in wässrigem Medium werden vorzugsweise auf 1 mMol Cyclodextrin 1–20 ml Wasser angewendet. Beim Arbeiten im wässrigen-äthanolischem Medium wird ein 5–32 Vol-%iges wässrig-äthanolisches Medium bevorzugt.

Wenn man die Cyclodextrine mit Phendilin in einem wässrigen und/oder äthanolischen Medium intensiv rührt, verdrängt das Phendilinmolekül die in dem inneren Teil des Hohlraums des Cyclodextrins vorhandenen Wassermoleküle und erzeugt eine apolare-apolare Gegenwirkung, wodurch der Inklusionskomplex entsteht.

Dieses mit Salzbildung kombinierte und von partieller Cyclodextrin-Eliminierung begleitete intensive Lösungsverfahren der mit Cyclodextrinen durchführbaren Moleküleinkapselung ist bislang nicht bekannt. Das wesentliche Merkmal der Erfindung besteht darin, dass das Gastmolekül in dem mit dem nicht-ionischen Gastmolekül gebildeten Cyclodextrin-Inklusionskomplex ionisiert wird, worauf infolge der ionischen Bindung eine grosse Ladungsintensität und ein hydratisierter Zustand erzeugt wird, mit der Folge, dass der Cyclodextrinüberzug vom Gastmolekül abgestossen wird. Wird der Inklusionskomplex eines in Wasser schwer löslichen, eine kristalline Gitterstruktur aufweisenden, nicht-ionischen Gastmoleküls in einen ionischen Zustand übergeführt, zerfällt das Kristallgranulat plötzlich und geht in einem molekulardispersen Zustand über, d. h. die ganze Menge löst sich auf.

Die so gebildete Molekülstruktur ist relativ gut löslich, weil das eine Ende des Moleküls infolge der Ionisierung hydratisiert ist und das andere Ende – das übrigens stark apolar und daher Ursache für die Schwerlöslichkeit des Moleküls ist – auf der Aussenseite mit einem hydrophilen Cyclodextrinring überzogen ist.

Phendilin ist in Wasser unlöslich, aber auch sein Hydrochlorid ist ausserordentlich schwer löslich. Wird die freie Base mit Salzsäure behandelt, so verhindert das auf der Oberfläche eines von der Base gebildeten Öltropfens entstandene stark hydrophobe Hydrochlorid die Lösung und sogar die weitere Salzbildung. Wenn ein Phendilinmolekül mit zwei Cyclodextrinmolekülen einen Inklusionskomplex bildet, wird eine in Wasser relativ schwer lösliche kristalline Substanz erhalten.

Kommt aber dieser Inklusionskomplex in ein saures Medium (z.B. in das der Magensäure) wird das Phendilin-Stickstoffatom durch Protonaufnahme ionisiert und stösst den in der Nähe des Stickstoffatoms befindlichen Cyclodextrinring ab. Hieraus resultiert eine Struktur, in der der ionische Phenyläthylamino-Teil des Phendilins frei und zum grössten Teil hydratisiert vorliegt und die apolare Diphenylpropylgruppe mit einem Molekül Cyclodextrin einen Komplex bildet. Das heisst, aus einem 2:1 Komplex entsteht im sauren Medium ein 1:1 Komplex, bzw. der Phendilin-Cyclodextrin-Komplex geht im sauren Zustand sehr schnell in einen molekular-dispersen Zustand über. Mit anderen Worten: der Komplex löst sich auf.

Die Cyclodextrinkomplexe des Phendilins bzw. seines Hydrochlorids lösen sich in Wasser besser als die entsprechenden komplexfreien Verbindungen. Der Komplex, in dem Cyclodextrin und die freie Base im molaren Verhältnis von 2:1 vorliegen, (durchschnittlicher Wirkstoffgehalt 11%) ist in Wasser besser löslich als Phendilin aber weniger löslich als das Phendilin-Hydrochlorid, und der Komplex, in dem Cyclodextrin und Phendilin-Hydrochlorid im molaren Verhältnis von 1:1 vorliegen (durchschnittlicher Wirkstoffgehalt 21%) ist in Wasser leichter löslich als Phendilin-Hydrochlorid. Der verbesserten Löslichkeit entspricht auch eine verstärkte biologische Aktivität der Komplexverbindungen, was für die Verwendung zur Herstellung von Medikamenten wichtig ist.

Zur Beurteilung der biologischen Wirksamkeit wurde unter in vitro-Bedingungen untersucht, wie weit das Auflösen und die Resorption des Phendilins bzw. des Phendilin-Hydrochlorids durch das Einschliessen des Wirkstoffes in einen Cyclodextrin-Inklusionskomplex beeinflusst wird.

Die Untersuchungen wurden unter in vitro-Bedingungen durchgeführt, weil sich diese Messungen besser reproduzieren lassen und die Streuung der Werte geringer ist, als wenn die Messungen unter in vivo-Bedingungen durchgeführt worden wären.

Das Auflösen und die Resorption sind aufeinander folgende Prozesse, die in starkem Masse gegenseitig voneinander abhängig sind. Unter physiologischen Bedingungen wird die Resorption einerseits von den an der Resorptionsstelle vorhandenen pH-Verhältnissen, andererseits von der Verweilzeit des Pharmakons an der Resorptionsstelle stark beeinflusst.

Die durchschnittliche Verweilzeit des Medikamentes und die pH-Werte in einigen Abschnitten des Magen-Darm-Traktes betragen:

| | |
|---|---|
| im Magen | 0– 60 Minuten, pH = 1,2 |
| im Ausgangsabschnitt des Dünndarms | 30– 60 Minuten, pH = 5–6 |
| in den weiteren Abschnitten des Dünndarmes | 60–360 Minuten, pH = 6–7 |

Dies wurde bei den Untersuchungen berücksichtigt.

Die Resorptions-Untersuchungen wurden mit einem Sartorius SM 16750 Resorptions Modell-Instrument durchgeführt, mit dem die Resorption aus dem Magen und dem Darm mit Hilfe der verwendeten Membranen sehr gut nachgeahmt werden kann. Auf Grund der erhaltenen Untersuchungsergebnisse wurden die Diffusions-($K_d$) und die Resorptionsgeschwindigkeitskonstante ($K_i$) auf die untersuchten Stoffe berechnet. Die Ergebnisse sind in der nachfolgenden Tabelle angegeben.

| Verbindung | $K_d$ ($cm^2 min^{-1}$) aus dem Darm | $K_i$ ($min^{-1}$) aus dem Darm |
|---|---|---|
| Phendilin | $6,9 \times 10^{-4}$ | $5,1 \times 10^{-3}$ |
| Phendilin-ß-Cyclodextrin-Komplex | $1,42 \times 10^{-3}$ | $1,24 \times 10^{-2}$ |
| Phendilinhydrochlorid | $6,7 \times 10^{-4}$ | $4,9 \times 10^{-3}$ |
| Phendilinhydrochlorid-ß-Cyclodextrin-Komplex | $1,71 \times 10^{-3}$ | $1,52 \times 10^{-2}$ |

Die Tabelle zeigt, dass bei der Resorption aus dem Darm die Werte von $K_d$ und $K_i$ um mehr als 100% durch die Komplexbildung gestiegen sind.

Die Bestimmung der gelösten ($M_G$) und resorbierten ($M_i$) Wirkstoffmenge wurde mit einem Löse-Modell-Instrument des Typs Sartorius SM 16751 durchgeführt. (H. Stricker: Pharm. Ind. 33, 157 (1971); H.Stricker: Drugs in Germany 14, 93 (1971)).

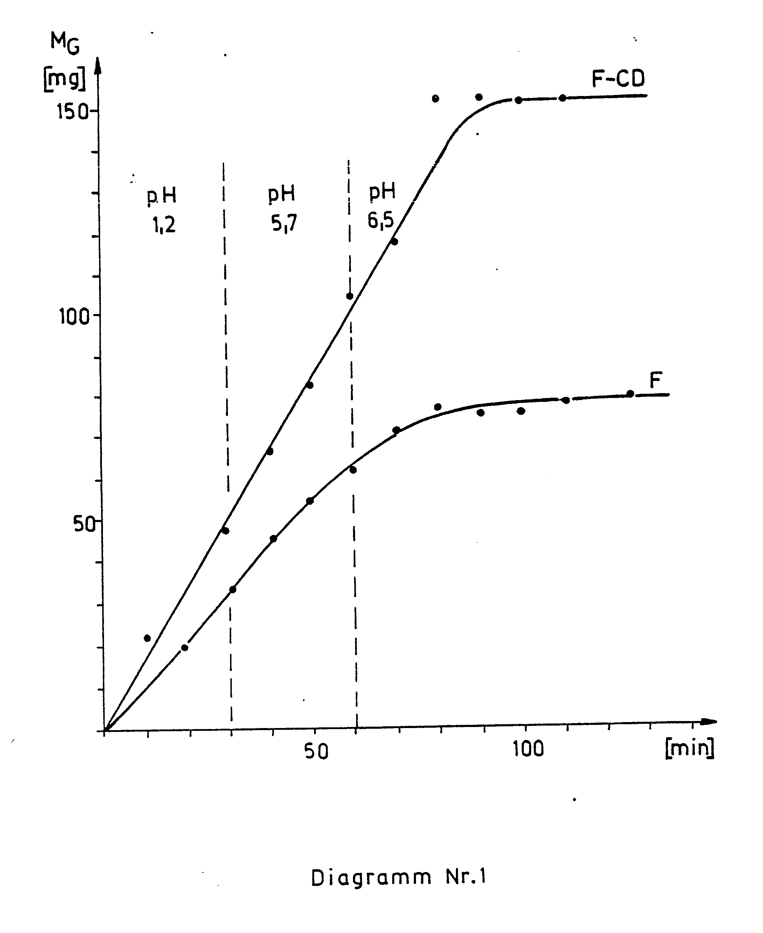

Die Zeitabhängigkeit der Auflösung des Phendilins und des Phendilin-Cyclodextrin-Komplexes in den einzelnen Abschnitten des Magen-Darm-Traktes ist im Diagramm Nr. 1 dargestellt, worin F die Auflösung des Phendilins und F–CD die Auflösung des Phendilin-Cyclodextrin-Komplexes bedeutet.

Die im Darm aufgelöste Menge Phendilin-Base beträgt nach hundert Minuten 75 mg, die des Phendilin-Cyclodextrin-Komplexes dagegen 150 mg.

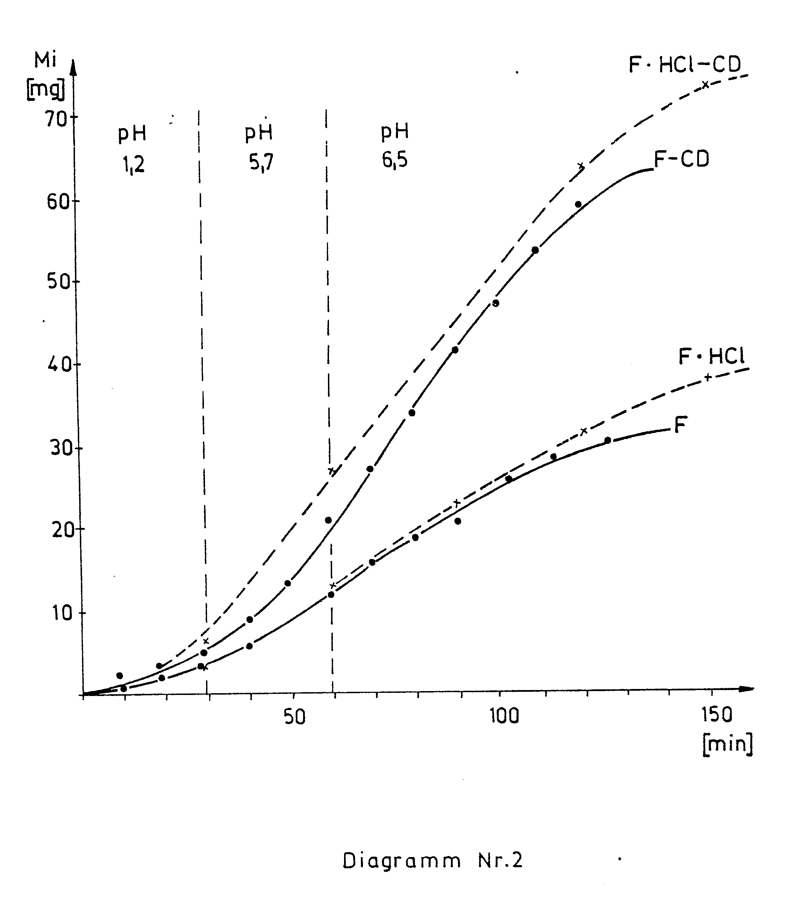

Die aus dem Magen-Darm-Trakt resorbierte Menge des Phendilins bzw. des Phendilin-Cyclodextrin-Komplexes und des Phendilin-Hydrochlorids bzw. des Phendilin-Hydrochlorid-Cyclodextrin-Komplexes ist in Diagramm Nr. 2 dargestellt, worin F die resorbierte Menge des Phendilins, F–CD die des Phendilin-Cyclodextrin-Komplexes, FHCl die des Phendilin-Hydrochlorid und FHCl–CD die des Phendilin-Hydrochlorid-Cyclodextrin-Komplexes bedeutet.

In Übereinstimmung mit den Auflösungswerten stieg nach der Komplexbildung die aus dem Darmtrakt resorbierte Wirkstoffmenge bei der Base sowie bei dem Salz auf fast das Doppelte. Dadurch ist es möglich, mit einer etwa halb so grossen Dosis die gleiche Wirkung zu erzielen.

Bei den toxikologischen Untersuchungen wurde die akute Toxizität des Phendilins bzw. des Phendilin-Cyclodextrin-Komplexes sowie des Phendilin-Hydrochlorids bzw. des Phendilin-Hydrochlorid-Cyclodextrin-Komplexes auf Mäuse intraperitoneal untersucht.

Die zu untersuchenden Stoffe wurden mit Hilfe von 5%igem Tween 80 je 10 männlichen Mäusen und 10 weiblichen Mäusen in jeder Gruppe gegeben. Die Dosis wurde bei den Komplexen auf den Phendilin-Wirkstoff berechnet. Die Resultate sind in der nachfolgenden Tabelle zusammengestellt.

Intraperitoneale Anwendung

|  | Beobachtungszeit | $LD_{50}$ (mg/kg) |
|---|---|---|
| Phendilin | 24 Stunden | 47,54 |
| Phendilin-Cyclodextrin-Komplex | 24 Stunden | 157,47 |
| Phendilin-Hydrochlorid | 1 Stunde | 81,14 |
| Phendilin-Hydrochlorid-Cyclodextrin-Komplex | 1 Stunde | 160,55 |
| Orale Anwendung |  |  |
| Phendilin | 72 Stunden | 512,41 |
| Phendilin-Cyclodextrin-Komplex | 72 Stunden | 624,65 |
| Phendilin-Hydrochlorid | 24 Stunden | 522,24 |
| Phendilin-Hydrochlorid-Cyclodextrin-Komplex | 24 Stunden | 954,88 |

Die Phendilin-Cyclodextrin-Komplexe der Erfindung sind weisse, pulverförmige, mikrokristalline Produkte, die als Wirkstoffe für pharmazeutische Präparate verwendet werden können. Diese Präparate enthalten ausser der wirksamen Menge des Cyclodextrin-Phendilin-Komplexes als Wirkstoff gegebenenfalls pharmazeutische anwendbare anorganische und organische Trägerstoffe.

Die Präparate können in Form von Tabletten, Dragées, Kapseln oder Syrup usw. verabreicht werden. Die Präparate enthalten den Wirkstoff vermischt mit Verdünnungsmitteln, Lactose, Dextrose, Saccharose, Glycin und/oder Gleitmitteln, z.B. Kieselgur. Talkum, Stearinsäure und deren Salzen, mit Polyäthylenglykol, Bindemitteln, Füllstoffen, Farbstoffen, den Geschmack verbessernden Mitteln usw.. Die Präparate können gegebenenfalls weitere biologisch aktive Wirkstoffe enthalten. Die Präparate können durch bekannte Tablettierungs-, Granulierungs- und Homogenisierungsmethoden hergestellt werden. Der Wirkstoffgehalt beträgt etwa 10–40%. Die Dosierung hängt von verschiedenen Faktoren, wie z.B. von der Art der Verabreichung, dem Zustand und Alter des Patienten ab.

Weitere Einzelheiten der Erfindung sind den nachstehenden Beispielen zu entnehmen.

Beispiel 1

10 g (7,5 mMol) 15% Wasser enthaltendes β-Cyclodextrin suspendiert man bei Raumtemperatur in 40 ml Wasser und tropft 0,96 g (3 mMol) Phendilin in 2 ml 96 Vol.%igem Äthanol unter intensivem Rühren zu. Die Suspension wird weitere 5 Stunden gerührt, filtriert und getrocknet.

Man erhält 9,2 g eines 9,7 Gew.% Phendilin enthaltenden Phendilin-β-Cyclodextrin-Komplexes. Das erhaltene Produkt stellt ein weisses Pulver dar, das keinen charakteristischen Schmelzpunkt hat.

Bestimmung des Phendilin-Gehalts des Komplexes: Man löst 0,05 g des Produktes in 25 ml 50 Vol.%igem Äthanol und führt die photometrische Bestimmung des Produktes bei 258 nm Wellenlänge gegen 50 Vol.%igen Äthanol durch. Der Phendilin-Gehalt wird mit Hilfe der Kalibrationskurve festgestellt.

Beispiel 2

Man löst 26,8 g (20 mMol) 15% Wasser enthaltendes β-Cyclodextrin in einem Gemisch von 350 ml Wasser und 50 ml 96 Vol.%igem Äthanol bei 60 °C und gibt innerhalb einer halben Stunde ein Gemisch von 3,15 g (10 mMol) Phendilin und 40 ml 96 Vol.%igem Äthanol unter intensivem Rühren zu. Das Gemisch wird innerhalb von 6 Stunden auf Raumtemperatur abgekühlt und über Nacht bei +4 °C stehen gelassen. Das ausgeschiedene kristalline Produkt wird filtriert und an der Luft getrocknet. Man erhält 28,4 g Phendilin-β-Cyclodextrin-Komplex, der 10,7 Gew.% Phendilin enthält.

Beispiel 3

Man suspensiert 13,3 g (10 mMol) 15% Wasser enthaltendes β-Cyclodextrin in 10 ml destilliertem Wasser in einer Reibschale, gibt eine Lösung von 1,55 g (5 mMol) Phendilin in 5 ml 96 Vol.%igem Äthanol zu, wobei man eine verdünnte Suspension erhält, die durch ständiges Triturieren homogenisiert wird. Nach etwa 30 Minuten hat die Suspension die Konsistenz einer Salbe, die in einem Exsikkator ausgebreitet und 24 Stunden über Phosphorpentoxid getrocknet wird. Der von Lösungsmittel und Wasser freie Komplex wird pulverisiert.

Man erhält 12,5 g Phendilin-β-Cyclodextrin-Komplex. Phendilin-Gehalt: 11,8 Gew.%.

Beispiel 4

Man löst 2,1 g (1,4 mMol) 15% Wasser enthaltendes γ-Cyclodextrin in 15 ml Wasser bei 40 °C und tropft unter Rühren ein Gemisch von 0,158 g (0,5 mMol) Phendilin in 1 ml 96 Vol.%igem Äthanol zu. Die Kristallabscheidung setzt schon während der Zugabe ein. Die Suspension wird innerhalb von 2 Stunden auf Raumtemperatur abgekühlt und über Nacht bei +4 °C stehen gelassen. Nach Filtrieren und Trocknen an der Luft erhält man 0,66 g Phendilin-γ-Cyclodextrin-Komplex, Phendilingehalt: 11,3 Gew.%.

Beispiel 5

1,0 g (1,0 mMol) 3,3% Wasser enthaltendes α-Cyclodextrin löst man bei 40 °C in 7 ml destilliertem Wasser. Unter Rühren tropft man ein Gemisch von 0,158 g (0,5 mMol) Phendilin in 1 ml 96 Vol.%igem Äthanol zu, wobei sich während der Zugabe die ersten Kristalle abscheiden. Die Suspension wird innerhalb von 2 Stunden auf Raumtemperatur abgekühlt und über Nacht bei +4 °C stehen gelassen. Das Gemisch wird filtriert und getrocknet.

Man erhält 0,5 g Phendilin-α-Cyclodextrin-Komplex mit einem Phendilingehalt von 12,2 Gew.%.

Beispiel 6

Man löst 1,8 g eines 11% Wasser enthaltenen Cyclodextrin-Gemisches (Zusammensetzung auf den Trockensubstanzgehalt bezogen, nach der HPLC chromatographischen Analyse: 70% β-Cyclodextrin, 20% γ-Cyclodextrin, 10% α-Cyclodextrin, durchschnittliches Molgewicht: 1151, mMol-Zahl: 1,4) in einem Gemisch von 20 ml destilliertem Wasser und 3 ml 96 Vol%igem Äthanol bei 60 °C und tropft unter intensivem Rühren ein Gemisch von 0,22 g (0,7 mMol) Phendilin in 2 ml 96 Vol-%igem Äthanol zu. Das erhaltene Gemisch wird innerhalb von 3 Stunden auf Raumtemperatur gekühlt und über Nacht bei +4 °C stehen gelassen. Das ausgefallene kristalline Produkt wird abfiltriert und an der Luft getrocknet.

Man erhält 1,3 g des Phendilin-Cyclodextrin-Komplexes, Phendilingehalt 11,0 Gew.%.

Beispiel 7

Man löst 4 (3 mMol) β-Cyclodextrin mit 15% Wassergehalt in 35 ml Wasser bei 60 °C und tropft unter Rühren 1,05 g (3 mMol) Phendilin-Hydrochlorid, gelöst in 8 ml 96 Vol-%igem Äthanol, zu. Das Gemisch wird innerhalb von 5 Stunden auf Raumtemperatur abgekühlt und über Nacht bei +4 °C stehen gelassen. Die ausgefallene kristalline weisse Substanz wird abfiltriert und getrocknet.

Man erhält 3,8 g des lufttrockenen Phendilin-Hydrochlorid-β-Cyclodextrin-Komplex, Phendilin-Hydrochloridgehalt 22,2 Gew.%.

Beispiel 8

Man löst 13,4 g (10 mMol) 15% Wasser enthaltendes β-Cyclodextrin in einem Gemisch von 160 ml Wasser und 10 ml 1 N Salzsäure bei 50 °C und gibt eine Lösung von 3,15 g (10 mMol) Phendilin in 30 ml 96 Vol-%igem Äthanol zu. Das Gemisch wird innerhalb von 4 Stunden auf Raumtemperatur abgekühlt und über Nacht bei +4 °C stehen gelassen. Die ausgefallene kristalline Substanz wird abfiltriert und getrocknet.

Man erhält 11,2 g Phendilin-Hydrochlorid-β-Cyclodextrin-Komplex mit einem Phendilin-Hydrochloridgehalt von 20,5 Gew.%.

Beispiel 9

5,0 g des nach Beispiel 2 hergestellten Phendilin-β-Cyclodextrin-Komplexes (Phendilingehalt: 10,7 Gew.%) werden in einem Gemisch von 35 ml Wasser und 2 ml 1 N Salzsäure bei 37 °C gelöst. Die Lösung wird 24 Stunden stehen gelassen und die ausgefallene kristalline Substanz abfiltriert.

Man erhält 2,0 g lufttrockenen Phendilin-Hydrochlorid-β-Cyclodextrin-Komplex mit 21,2 Gew.% Phendilin-Hydrochloridgehalt.

Beispiel 10

Nachweis der Komplexstruktur des nach Beispiel 1 hergestellten Produktes durch Röntgendiffraktion

Die charakteristischen Reflexionsspitzen erscheinen bei signifikant verschiedenen 2 °C Winkelwerten an den Röntgendiffraktions-Pulveraufnahmen des β-Cyclodextrins und des Phendilin-β-Cyclodextrins, und diese Tatsache weist auf die verschiedenen Kristallstrukturen hin. Da das in dem Komplex eingeschlossene Molekül flüssig ist, bestätigt die unterschiedliche Kristallstruktur die Komplexbildung.

Untersuchung des nach Beispiel 1 hergestellten Produktes durch thermoanalytische Methoden

Die thermoanalytischen Untersuchungen zeigen wesentliche Unterschiede zwischen dem β-Cyclodextrin-Phendilin-Komplex und dem physikalischen Gemisch von Phendilin und β-Cyclodextrin. Das Verdampfen des Phendilins bzw. dessen Zersetzung beginnt bei 150 °C und bis 250 °C kann eine 98%ige Massenveränderung beobachtet werden. Cyclodextrin zersetzt sich erst ab 270 °C, und bei 300 °C schmilzt des β-Cyclodextrin unter Zersetzung.

Das physikalische Gemisch verliert bei 100 °C das Wasser des Cyclodextrins und im Bereich von 150 bis 250 °C das Phendilin.

Der Wirkstoff wird aus dem Komplex nur bei der Zersetzung des Cyclodextrins freigesetzt, also bei 270–300 °C. Die thermoanalytischen Untersuchungen zeigen, dass Phendilin mit β-Cyclodextrin im molaren Verhältnis von 1:2 einen Komplex bildet. Bei einem Überschuss an Phendilin verhält sich der Phendilin-Überschuss wie in einem physikalischen Gemisch, d.h. die Zersetzung beginnt schon bei 150–250 °C.

Beispiel 11
Untersuchung der Löslichkeit des nach Beispiel 7 hergestellten Phendilin-Hydrochlorid-β-Cyclodextrin-Komplexes

Man löst in 10 ml Wasser bei 37 °C Phendilin-Hydrochlorid-β-Cyclodextrin-Komplex in einer Menge, die 200 mg Phendilin-Wirkstoff entspricht, die Lösung wird bei 150 U/Min. mit einem magnetischen Rührer gerührt. Der Wirkstoffgehalt der Proben wird spektrophotometrisch bestimmt.

Die Versuchsergebnisse bezüglich der Lösungsgeschwindigkeit sind in der nachstehenden Tabelle zusammengestellt:

| Zeit | Phendilin-Hydrochlorid | Phendilin-Hydrochlorid β-Cyclodextrin-Komplex |
|---|---|---|
| Minuten | Phendilin-Konzentration mg/ml | |
| 1 | 1,2 | 13,3 |
| 5 | 2,5 | 12,7 |
| 15 | 2,4 | 12,9 |
| 30 | 2,5 | 12,8 |
| 60 | 2,6 | 13,3 |
| 120 | – | 14,6 |

Untersuchung der Zusammensetzung des nach Beispiel 7 hergestellten Phendilin-Hydrochlorid-β-Cyclodextrin-Komplexes

Man rührt 0,22 g (0,6 mMol) Phendilin-Hydrochlorid in 3 ml Wasser bzw. in β-Cyclodextrinlösungen verschiedener Konzentrationen bei 37 °C 3 Stunden mit einem magnetischen Rühren mit 150 U/Min.. Die Konzentration des gelösten Phendilin-Hydrochlorid wird spektrophotometrisch bestimmt und in Phendilin-Äquivalenten ausgedrückt. Die Versuchsergebnisse sind in der folgenden Tabelle zusammengestellt.

| β-Cyclodextrin-Konzentration | | Phendilin-Konzentration | | β-Cyclodextrin mMol Phendilin mMol |
|---|---|---|---|---|
| mg/ml | mMol/l | mg/ml | mMol/l | |
| 15 | 13,2 | 4,39 | 13,9 | 0,95 |
| 20 | 17,6 | 5,95 | 18,6 | 0,95 |
| 25 | 22,0 | 6,94 | 22,0 | 1,00 |
| 30 | 26,4 | 7,51 | 23,8 | 1,10 |

Der Wert des β-Cyclodextrin Mol/Phendilin Mol-Quotienten in der letzten Spalte der Tabelle zeigt eindeutig, dass Phendilin-Hydrochlorid mit β-Cyclodextrin praktisch einen 1:1 molaren Komplex bildet.

Beispiel 12
Herstellung eines tablettierten Präparates aus dem nach Beispiel 3 erhaltenen Phendilin-β-Cyclodextrin-Komplex

| | |
|---|---|
| Phendilin-β-Cyclodextrin-Komplex | 398 g |
| Magnesiumstearat | 5 g |
| kolloidale Kieselsäure | 7 g |
| mikrokristalline Cellulose (Avicel PH 102) | 90 g |

Die Komponenten werden miteinander vermischt, homogenisiert und zu Tabletten mit einem Durchschnittsgewicht von 500 mg und einem Durchmesser von 13 mm verpresst.

Die Tabletten enthalten durchschnittlich 45 mg Phendilin, was 50 mg Phendilin-Hydrochlorid entspricht. Eine Tablette enthält also die äquivalente Wirkstoffmenge wie eine 50 mg Phendilin-Hydrochlorid enthaltende Tablette.

Beispiel 13
Herstellung eines Syrups aus dem nach Beispiel 3 erhaltenen Phendilin-β-Cyclodextrin-Komplex

| | |
|---|---|
| Phendilin-β-Cyclodextrin-Komplex | 5 g |
| Methyl-p-oxybenzoat | 0,1 g |
| Aromastoff | 0,5 g |
| Carboxymethylcellulose-Natriumsalz | 0,9 g |
| mikrokristalline Cellulose (Avicel RC) | 1,1 g |
| Sorbit | 35 g |
| destilliertes Wasser | ad 100 ml |

Das Natriumsalz der Carboxymethylcellulose löst man in etwa der halben Menge Wasser, suspendiert die mikrokristalline Cellulose in der Lösung und mischt der Suspension nach dem Quellen den Phendilin-β-Cyclodextrin-Komplex zu. Methyl-p-oxybenzoat, der Aromastoff und der Sorbit werden in dem restlichen Wasser gelöst und die so erhaltene Lösung der obigen Suspension zugegeben. Das Volumen der Suspension wird mit destilliertem Wasser auf 100 ml ergänzt und die Suspension homogenisiert.

**Patentansprüche für die Vertragsstaaten; CH/LI, DE, FR, GB**

1. Inklusionskomplex von N-(1-Phenyläthyl)-3,3-diphenylpropylamin oder dessen Hydrochlorid und Cyclodextrin.

2. Inklusionskomplex nach Anspruch 1, dadurch gekennzeichnet, dass er als Cyclodextrin α-, β- oder γ-Cyclodextrin enthält.

3. N-(1-Phenyläthyl)-3,3-diphenylpropylamin-β-Cyclodextrin-Komplex.

4. N-(1-Phenyläthyl)-3,3-diphenylpropylamin-Hydrochlorid-β-Cyclodextrin-Komplex.

5. Verfahren zur Herstellung von Inklusionskomplexen von N-(1-Phenyläthyl)-3,3-diphenylpropylamin oder dessen Hydrochlorid mit Cyclodextrin, dadurch gekennzeichnet, dass man N-(1-Phenyläthyl)-3,3-diphenylpropylamin-Base oder deren Hydrochlorid im wässrigen und/oder äthanolischen Medium unter Rühren bei 4 bis 60 °C mit Cyclodextrin im Verhältnis von 1–3 mMol Cyclodextrin pro 1 mMol Base oder Hydrochlorid umsetzt, wobei der mit der Base erhaltene Cyclodextrin-Komplex nachträglich durch Behandeln mit Salzsäure in den Hydrochlorid-Komplex übergeführt werden kann.

6. Verfahren nach Anspruch 5, dadurch gekennzeichnet, dass man N-(1-Phenyläthyl)-3,3-diphenylpropylamin mit Cyclodextrin im Verhältnis von 1:2 umsetzt.

7. Verfahren nach Anspruch 5, dadurch gekennzeichnet, dass man N-(1-Phenyläthyl)-3,3-diphenylpropylamin-Hydrochlorid mit Cyclodextrin im Verhältnis von 1:1 umsetzt.

8. Verfahren nach Anspruch 5, dadurch gekennzeichnet, dass man die Komplexbildung mit dem in situ gebildeten Hydrochlorid des N-(1-Phenyläthyl)-3,3-diphenylpropylamins durchführt.

9. Verfahren nach Ansprüchen 5, 6, 7 oder 8, dadurch gekennzeichnet, dass man als Cyclodextrin α-, β- oder γ-Cyclodextrin verwendet.

10. Pharmazeutische Präparate, die als Wirkstoff einen Cyclodextrin-Inklusionskomplex der Ansprüche 1 bis 4 enthalten.

**Patentansprüche für den Vertragsstaat: AT**

1. Verfahren zur Herstellung von Inklusionskomplexen von N-(1-Phenyläthyl)-3,3-diphenylpropylamin oder dessen Hydrochlorid mit Cyclodextrin, dadurch gekennzeichnet, dass man N-(1-Phenyläthyl)-3,3-diphenylpropylamin-Base oder deren Hydrochlorid im wässrigen und/oder äthanolischem Medium unter Rühren bei 4 bis 60 °C mit Cyclodextrin im Verhältnis von 1–3 mMol Cyclodextrin pro 1 mMol Base oder Hydrochlorid umsetzt, wobei der mit der Base erhaltene Cyclodextrin-Komplex nachträglich durch Behandeln mit Salzsäure in den Hydrochlorid-Komplex übergeführt werden kann.

2. Verfahren nach Anspruch 1, dadurch gekennzeichnet, dass man N-(1-Phenyläthyl)-3,3-diphenylpropylamin mit Cyclodextrin im Verhältnis von 1:2 umsetzt.

3. Verfahren nach Anspruch 1, dadurch gekennzeichnet, dass man
N-(1-Phenyläthyl)-3,3-diphenylpropylamin-Hydrochlorid
mit Cyclodextrin im Verhältnis von 1:1 umsetzt.

4. Verfahren nach Anspruch 1, dadurch gekennzeichnet, dass man die Komplexbildung mit dem in situ gebildeten Hydrochlorid des N-(1-Phenyläthyl)-3,3-diphenylpropylamins durchführt.

5. Verfahren nach Ansprüchen 1, 2, 3 oder 4, dadurch gekennzeichnet, dass man als Cyclodextrin α-, β- oder γ-Cyclodextrin verwendet.

6. Verwendung von Cyclodextrin-Inklusionskomplexen aus N-(1-Phenyläthyl)-3,3-diphenylpropylamin oder dessen Hydrochlorid und α-, β- oder γ-Cyclodextrin als Wirkstoff zur Herstellung von pharmazeutischen Präparaten.

**Claims for the Contracting States: CH, DE, FR, GB, LI**

1. An inclusion complex of N-(1-phenylethyl)-3,3-diphenylpropylamine or its hydrochloride and cyclodextrin.

2. An inclusion complex according to Claim 1, characterized in that it contains α-, β- or γ-cyclodextrin as the cyclodextrin.

3. N-(1-Phenylethyl)-3,3-diphenylpropylamine-β-cyclodextrin complex.

4. N-(1-Phenylethyl)-3,3-diphenylpropylamine-hydrochloride-β-cyclodextrin complex.

5. A process for the preparation of inclusion complexes of N-(1-phenylethyl)-3,3-diphenylpropylamine or its hydrochloride with cyclodextrin, characterized in that N-(1-phenylethyl)-3,3-diphenylpropylamine base or its hydrochloride in aqueous and/or ethanolic medium is reacted with cyclodextrin in a ratio of 1–3 mMol cyclodextrin per 1 mMol base or hydrochloride with stirring at 4–60 °C, whereby the cyclodextrin complex obtained with base may subsequently be converted to the hydrochloride complex by treatment with hycrochloric acid.

6. A process according to Claim 5, characterized in that N-(1-phenylethyl)-3,3-diphenylpropylamine is reacted with cyclodextrin in a ratio of 1:2.

7. A process according to Claim 5, characterized in that N-(1-phenylethyl)-3,3-diphenylpropylamine hydrochloride is reacted with cyclodextrin in a ratio of 1:1.

8. A process according to Claim 5, characterized in that formation of the complex is carried out with the in situ formed hydrochloride of N-(1-phenylethyl)-3,3-diphenylpropylamine.

9. A process according to Claims 5, 6, 7 or 8, characterized in that α-, β- or γ-cyclodextrin is used as the cyclodextrin.

10. Pharmaceutical products that contain a cyclodextrin inclusion complex according to Claims 1–4 as the active agent.

### Claims for the Contracting State: AT

1. A process for the preparation of inclusion complexes of N-(1-phenylethyl)-3,3-diphenylpropylamine or its hydrochloride with cyclodextrin, characterized in that N-(1-phenylethyl)-3,3-diphenylpropylamine base or its hydrochloride in aqueous and/or ethanolic medium is reacted with cyclodextrin in a ratio of 1–3 mMol cyclodextrin per 1 mMol base or hydrochloride with stirring at 4–60 °C, whereby the cyclodextrin complex obtained with base may subsequently be converted to the hydrochloride complex by treatment with hydrochloric acid.

2. A process according to Claim 1, characterized in that N-(1-phenylethyl)-3,3-diphenylpropylamine is reacted with cyclodextrin in a ratio of 1:2.

3. A process according to Claim 1, characterized in that N-(1-phenylethyl)-3,3-diphenylpropylamine hydrochloride is reacted with cyclodextrin in a ratio of 1:1.

4. A process according to Claim 1, characterized in that formation of the complex is carried out with the in situ formed hydrochloride of N-(1-phenylethyl)-3,3-diphenylpropylamine.

5. A process according to Claims 1, 2, 3, or 4, characterized in that α-, β- or γ-cyclodextrin is used as the cyclodextrin.

6. Use of cyclodextrin inclusion complexes of N-(1-phenylethyl)-3,3-diphenylpropylamine or its hydrochloride and α-, β- or γ-cyclodextrin as active agents for the preparation of pharmaceutical products.

### Revendications pour les Etats contractants CH, DE, FR, GB, LI

1. Complexe d'inclusion de N-(1-phényléthyl)-3,3-diphénylpropylamine ou de son chlorhydrate et de cyclodextrine.

2. Complexe d'inclusion selon la revendication 1, caractérisé en ce qu'il contient comme cyclodextrine l'α-, β- ou γ-cyclodextrine.

3. Complexe N-(1-phényléthyl)-3,3-diphénylpropylamine-β-cyclodextrine.

4. Complexe chlorhydrate de N-(1-phényléthyl)-3,3-diphénylpropylamine-β-cyclodextrine.

5. Procédé de préparation de complexes d'inclusion de N-(1-phényléthyl)-3,3-diphénylpropylamine ou de son chlorhydrate avec la cyclodextrine, caractérisé en ce qu'on fait réagir la N-(1-phényléthyl)-3,3-diphénylpropylamine base ou son chlorhydrate en milieu aqueux et/ou éthanolique tout en agitant à 4 à 60 °C avec de la cyclodextrine dans un rapport de 1–3 mMol de cyclodextrine pour 1 mMol de base ou de chlorhydrate, et en ce qu'on peut ensuite transformer le complexe de cyclodextrine obtenu avec la base par traitement avec de l'acide chlorhydrique en le complexe de chlorhydrate.

6. Procédé selon la revendication 5, caractérisé en ce qu'on fait réagir la N-(1-phényléthyl)-3,3-diphénylpropylamine avec la cyclodextrine dans un rapport de 1:2.

7. Procédé selon la revendication 5, caractérisé en ce qu'on fait réagir le chlorhydrate de N-(1-phényléthyl)-3,3-diphénylpropylamine avec la cyclodextrine dans un rapport de 1:1.

8. Procédé selon la revendication 5, caractérisé en ce qu'on effectue la formation de complexe avec le chlorhydrate formé in situ de la N-(1-phényléthyl)-3,3-diphénylpropylamine.

9. Procédé selon les revendications 5, 6, 7 ou 8, caractérisé en ce qu'on utilise comme cyclodextrine l'α-, β- ou γ-cyclodextrine.

10. Préparations pharmaceutiques qui contiennent comme substance active un complexe d'inclusion de cyclodextrine des revendications 1 à 4.

### Revendications pour l'Etat contractant AT

1. Procédé de préparation de complexes d'inclusion de N-(1-phényléthyl)-3,3-diphénylpropylamine ou de son chlorhydrate avec la cyclodextrine, caractérisé en ce qu'on fait réagir la N-(1-phényléthyl)-3,3-diphénylpropylamine base ou son chlorhydrate en milieu aqueux et/ou éthanolique tout en agitant à 4 à 60 °C avec de la cyclodextrine dans un rapport de 1–3 mMol de cyclodextrine pour 1 mMol de base ou de son chlorhydrate, où l'on peut ensuite transformer le complexe de cyclodextrine obtenu avec la base par traitement avec de l'acide chlorhydrique en le complexe de chlorhydrate.

2. Procédé selon la revendication 1, caractérisé en ce qu'on fait réagir la N-(1-phényléthyl)-3,3-diphénylpropylamine avec la cyclodextrine dans un rapport de 1:2.

3. Procédé selon la revendication 1, caractérisé en ce qu'on fait réagir le chlorhydrate de N-(1-phényléthyl)-3,3-diphénylpropylamine avec la cyclodextrine dans un rapport de 1:1.

4. Procédé selon la revendication 1, caractérisé en ce qu'on effectue la formation du complexe avec le chlorhydrate formé in situ de la N-(1-phényléthyl)-3,3-diphénylpropylamine.

5. Procédé selon les revendications 1, 2, 3 ou 4, caractérisé en ce qu'on utilise comme cyclodextrine l'$\alpha$-, $\beta$- ou $\gamma$-cyclodextrine.

6. Application de complexes d'inclusion de cyclodextrine de N-(1-phényléthy)-3,3-diphénylpropylamine ou de son chlorhydrate et d'$\alpha$-, $\beta$- ou $\gamma$-cyclodextrine comme substance active pour la préparation de préparations pharmaceutiques.

0 078 820

Diagramm Nr.1

11

Diagramm Nr.2